Europäisches Patentamt

**European Patent Office**

**Office européen des brevets**

(11) Publication number: **0 133 599**
**A2**

# (12) EUROPEAN PATENT APPLICATION

(21) Application number: **84305434.7**

(22) Date of filing: **09.08.84**

(51) Int. Cl.⁴: **A 61 K 39/29**

(30) Priority: **12.08.83 GB 8321789**

(43) Date of publication of application:
**27.02.85 Bulletin 85/9**

(84) Designated Contracting States:
**AT BE CH DE FR GB IT LI LU NL SE**

(71) Applicant: **BIOGEN N.V.**
**15 Pietermaai**
**Willemstad Curacao, Netherlands Antilles(NL)**

(72) Inventor: **Murray, Kenneth**
**4 Blackford Hill View**
**Edinburgh EH9 3HD Scotland(GB)**

(74) Representative: **Bannerman, David Gardner et al,**
**Withers & Rogers 4 Dyer's Buildings Holborn**
**London, EC1N 2JT(GB)**

(54) **Vaccines and compositions against hepatitis B viral infections.**

(57) Anti-HBe based vaccines and pharmaceutically acceptable compositions for treating patients against hepatitis B viral infections. The vaccines and compositions are characterized by a component that elicits in a treated patient the formation of anti-HBe at a titer effective to protect the patient for some period of time against hepatitis B viral infection or at a titer effective to lessen the severity of a hepatitis B viral infection in that patient.

EP 0 133 599 A2

0133599

B51

# VACCINES AND COMPOSITIONS AGAINST HEPATITIS B VIRAL INFECTIONS

## TECHNICAL FIELD OF INVENTION

This invention relates to vaccines and compositions against hepatitis B viral infections. This invention relates more particularly to vaccines and to other pharmaceutically acceptable compositions that are characterized by a component that elicits in the treated patient the formation of antibodies to hepatitis B virus e antigen ("anti-HBe") at a titer effective to protect against, or at least to lessen the severity of, a hepatitis B viral infection in that patient. It also relates to compositions and methods that are characterized by components that elicit in the treated patient the formation of antibodies to hepatitis B virus core antigens ("anti-HBc"), as well as the above-described anti-HBes. As will be appreciated from the disclosure to follow the vaccines and compositions of this invention, and the methods of using them, are useful in treating humans against hepatitis B viral infections and in protecting them for some period of time against those infections.

## BACKGROUND ART

Hepatitis B virus ("HBV") infects humans at a very high rate. The disease affects some 200 million people worldwide. For example, it is estimated that 4% of the United States population have been infected, and in some African and Asian countries, as many as 10% of the adult population are contagious, chronic HBV carriers, with over 15% infected.

HBV infection is transmitted by three general mechanisms: (1) by inoculation with infected blood or body fluids, either in large amounts (as in blood transfusions) or in small amounts (as in an accidental skinprick); (2) by close family or sexual contact; and (3) by infection during pregnancy, where the mother transmits the virus to her child.

Most HBV infections are subclinical, and recovery from both subclinical and clinical infections is usually complete. However, serious long-term consequences occur in some cases: (1) about 5% of acute HBV infections result in chronic HBV infection, with the constant potential for infectivity to others and for serious, debilitating liver disease, and (2) it is likely that past infection with HBV may be partly or even wholly responsible for the initiation of fulminant hepatitis, cirrhosis, and primary liver cancer. For example, the normal incidence of primary liver cancer is 1:100,000, but for chronic HBV sufferers the incidence of that cancer is 1:300.

The widespread occurrence of HBV, together with its virulence and its association with chronic or lethal liver disease, constitutes a clinical problem of considerable importance. At constant risk are: (1) blood recipients, patients undergoing hemodialysis or renal dialysis, and the institutionalized; (2) their families and (3) all health professionals (particularly nurses, surgeons and dentists). Hence, it is of paramount importance that vaccines be made available to protect these high risk individuals, as well as to protect the general populace, at least for some period of time, from hepatitis B viral infections.

It is believed that the Dane particle is identical or closely related to the infective virion of HBV [D. S. Dane and C. H. Cameron, "Virus-Like Particles In Serum Of Patients With Australia-Antigen Associated Hepatitis", The Lancet, 1, pp. 695-98 (1970)]. Generally, the Dane particle is believed to consist of an outer layer and an inner core. The outer layer of the particle generally contains a polypeptide known as hepatitis B surface antigen ("HBsAg"). The inner core (27 nanometers in diameter) contains a second polypeptide, hepatitis B core antigen ("HBcAg"), as well as an endogenous doublestranded, circular, DNA molecule of $2.1 \times 10^6$ daltons, which contains a single-stranded gap of varying length. The Dane particle is also believed to include an endogenous DNA dependent DNA polymerase that partially closes the single-stranded gap in the endogenous DNA by polymerase reaction employing that DNA as a primer/template.

A third polypeptide, hepatitis B e antigen ("HBeAg"), may also be associated with the Dane particle. For example, HBeAg has been variously attributed to the DNA polymerase enzyme of HBV [J. L. Melnick et al., "Approaching The Control Of Viral Hepatitis Type B", J. Infectious Diseases, 133, pp. 210-25 (1976)], an idiotype of IgG [A. R. Neurath and N. Strick, "Host Specificity Of A Serum Marker For Hepatitis B: Evidence That Virus e Antigen Has The Properties Of An Immunoglobulin", Proc. Natl. Acad. Sci. (USA), 74, pp. 1702-06 (1977)], a dimer of IgG associated with a small peptide [H. A. Fields et al., "Purification And Partial Characterization Of Hepatitis e Antigen",

*Infection & Immunity*, 20, pp. 792-803 (1978)], a component associated with lactate dehydrogenase isoenzyme no. 5 [G. N. Vyas et al., "Hepatitis B Virus e Antigen: An Apparent Association With Lactate Dehydrogenase Isoenzyme 5", *Science*, 198, pp. 1068-70 (1977)], or an antigenic marker on the surface of Dane particles and tubular forms [Neurath et al., "Identification Of Additional Antigenic Sites On Dane Particles And The Tubular Form Of Hepatitis B Surface Antigen", *J. Gen Virol.*, 30, pp. 277-85 (1976). More recently, HBeAg has been demonstrated to be derived from HBcAg [European patent application 75,395].

It is known that HBV infections cause development of antibodies to the antigens of the virus. See, e.g., Figure 3, European patent application 75,395. One of these antigens, hepatitis B virus surface antigen ("HBsAg"), is believed to be responsible for causing the development of antibodies (anti-HBs) that are protective against HBV infection. The other antigens of HBV (HBcAg and HBeAg) are believed to elicit the formation of antibodies (anti-HBe and anti-HBe, respectively) that are not protective against HBV infection. For example, no detectable quantities of HBcAg are stated to be present in the HBV antigenic compositions referred to in European patent 5864. In fact, specific efforts are taken to remove HBcAg from that antigenic composition. In like manner, HBeAg is said in that European patent to provide to HBsAg-based antigenic compositions only additional protection against HBV reinfection. And, HBeAg is said in United States patent 4,118,479 only to supplement the protective effect of anti-HBs on exposure of a patient to large doses of HBV. See also Neurath et al. (1976), *supra*. Accordingly, numerous and long-standing efforts have been made to develop HBV vaccines based on HBsAg.

However, these efforts have been hampered by the limited host range of HBV. For example, although highly infectious to man, experimental infection with hepatitis B virus has been achieved in only a few additional primates, most notably chimpanzees. The virus is also difficult to propagate or to attenuate in tissue culture [see United States patent 4,301,250]. Accordingly, presently-available vaccines against HBV are made from HBsAg's extracted from serum of human carriers of the disease [see, e.g., United States patents 3,636,191, 4,017,360 and 4,301,250].

This source of HBsAg, as well as the techniques used to isolate HBsAg from it, make production of a vaccine based on native HBsAg time-consuming and expensive. For example, the serum is infectious. Therefore, all work with it must be done in isolated areas by workers who are immune to the disease. In addition, each batch of the serum-derived HBsAg vaccine must be carefully tested in chimpanzees to insure that no HBV infectious agents are present. There is also a limited supply of the necessary HBsAg serum. Accordingly, a vaccine based on HBsAg that is derived from infected serum is available only in limited supply and at a cost too high to permit treatment of other than very high risk individuals. In addition to those disadvantages of supply and cost, serum-derived vaccines are also disadvantaged by the general reticence among patients against treatment with live virus-derived vaccines because of the possibility of infection and more particularly against treatment with serum-derived compounds because of the potential, however, small, for infection by other serum-derived contaminants therein. This is of particular concern at present because the vaccines require high titers of plasma, much of which comes from infected individuals who have a high incidence of venereal disease and acquired immune deficiency disease ("AIDS"). Finally, certain members of the populace are not sensitive to serum-derived HBsAg vaccines. In particular, 20 to 30% of dialysis patients, who are at high risk to hepatitis B viral infection, cannot be protected with that vaccine.

In an attempt to avoid some of the disadvantages inherent in serum-derived HBsAg vaccines against HBV, the methodology of recombinant DNA technology has been employed to produce HBsAg in a variety of hosts. E.g., European patent application 13828.

Although recombinant-produced HBsAg has been shown to elicit antibodies (anti-HBs) against hepatitis B viral infection in chimpanzees and to protect those chimpanzees against HBV infection [e.g., McGraw-Hill's Biotechnology Newswatch, 3, pp. 1-2 (August 1, 1983)], vaccines based on these products are still somewhat disadvantaged for use by the general populace. For example, the expression levels of DNA sequences encoding HBsAg are lower than desired in most hosts. In addition, the level of immunogenic activity of the produced HBsAg seems to depend to some extent on the particular host employed. For example, genetically-engineered yeast appears to be among the more favorable strains for producing immunogenic compositions. However, such yeast strains do not at present produce as much HBsAg as desired. Moreover, the isolation and purification of HBsAg from those yeast strains is comparatively difficult and time consuming. Moreover, although these recombinant HBsAg vaccines cannot accidentally infect a treated patient, they do not avoid the aforementioned insensitivity of certain patients, especially those on dialysis, to HBsAg. Accordingly, other sources of vaccines against hepatitis B viral infection are required.

## DISCLOSURE OF THE INVENTION

The present invention solves the problems referred to by providing vaccines and other pharmaceutically acceptable compositions that are characterized by a component that elicits in a treated patient the formation of antibodies to hepatitis B viral e antigens at a titer effective to protect the patient against, or at least to lessen the severity of, a hepatitis B viral infection. These vaccines and compositions, as well as methods of using them, are useful in treating humans against hepatitis B viral infections and in protecting them for some period of time against those infections.

By virtue of our invention, it is now unexpectedly, and for the first time possible, to protect humans at least for some period of time against hepatitis B viral infection, or at least to lessen the severity, in terms of time and virulence, of that infection, by treating those humans in a pharmaceutically effective manner with a vaccine or composition characterized by a component that elicits the formation of antibodies to hepatitis B viral e antigens (anti-HBe) at a titer effective to protect the human from HBV infection. The methods and compositions of this invention may also be characterized by components that elicit in the treated patient the formation of antibodies to hepatitis B virus core antigens as well as the above-described anti-HBes. Moreover, it is another advantage of this invention, that no HBsAg need be present in these vaccines and compositions.

## BRIEF DESCRIPTION OF THE DRAWINGS

Figure 1 displays the serum conversion analyses (HBsAg, HBeAg, anti-HBs, anti-HBe and anti-HBc) for serum samples taken weekly from chimpanzees Gwen and Rinka, who were inoculated with a composition in accordance with this invention and challenged by an intravenous inoculation of HBV serotype ayw.

Figure 2 displays the serum conversion analyses (HBsAg, HBeAg, anti-HBs, anti-HBe and anti-HBc) for serum samples taken weekly from control chimpanzees Peter and Frankje, who were not treated in accordance with this invention, but who were challenged by an intravenous inoculation of HBV serotype ayw.

Figure 3 displays the serum conversion analyses (HBeAg, anti-HBs, anti-HBe and anti-HBc) for serum samples taken weekly from chimpanzees A and B treated with compositions in accordance with this invention and challenged by intravenous inoculation of HBV serotype ayw.

### BEST MODE OF CARRYING OUT THE INVENTION

In order that the invention herein described may be more fully understood, the following detailed description is set forth.

In this description the following terms are employed:

Polypeptide: A linear series of amino acids connected one to the other by peptide bonds between the $\alpha$-amino and carboxy groups of adjacent amino acids.

Protein: A polypeptide with approximately 50 or more amino acids.

Antibody: A protein produced by animals in response to the presence of a foreign protein. The antibody binds very strongly and specifically to the foreign protein.

Antigen: Any polypeptide or protein some part or parts of which can be bound by an antibody or antibodies.

Serum: That fraction of blood which remains after red blood cells are removed. It contains, inter alia, antibodies and antigens.

DNA Sequence: A linear series of nucleotides connected one to the other by phosphodiester bonds between the 3' and 5' carbons of adjacent pentoses.

Expression: The process undergone by a gene to produce a polypeptide or protein. It is a combination of transcription and translation.

Expression Control Sequence: A DNA sequence that controls and regulates expression of genes when operatively linked to those genes.

Cloning Vehicle or Plasmid:. A DNA sequence which is able to reproduce itself in a host cell, characterized by one or a small number of endonuclease recognition sites at which such DNA sequences may be cut in a determinable fashion without attendant loss of an essential biological function of the DNA, and which contains a marker, either before or after transformation, suitable for use in the identification of transformed cells, e.g., tetracycline resistance or ampicillin resistance.

Recombinant DNA Molecule: A hybrid DNA sequence comprising at least two nucleotide sequences, the first sequence not normally being found together in nature with the second.

As described in this application, the vaccines, pharmaceutically acceptable compositions and methods of this invention are characterized by a component that elicits in a treated patient the formation of anti-HBe at a titer effective to protect that patient, for at least some period of time, from hepatitis B viral infection, or at least at a titer effective to lessen the severity, in terms of time and virulence, of any HBV infection.

The anti-HBe eliciting components that characterize the anti-HBe based vaccines and compositions of this invention may be selected from a wide variety of available sources. For example, they may be selected from a wide variety of pharmaceutically acceptable compositions that contain from 100% down to the lowest concentration of any of the various serological variants of HBeAg that is effective after one or more treatments of a patient in eliciting the required anti-HBe titer in that patient.

These anti-HBe eliciting components may also be derived from compositions containing precursors of any of the serological variants of HBeAg. In this latter case, it should be understood that the conversion of the precursor(s) to the desired amount of an HBeAg must be done in a pharmaceutically acceptable manner and without the co-production of pharmaceutically unacceptable by-products. It should also be understood, that although this conversion is preferably carried out prior to treatment of a patient with the composition or vaccine of this invention, the conversion may also be carried out in the patient as a result of *in vivo* metabolism. In that case the employed composition acts much like a pro-drug to produce the desired anti-HBe eliciting component in the patient as a metabolite.

Among the general methods useful for the conversion of these precursors of any of the serological variants of HBeAg to the desired anti-HBe eliciting components that characterize the vaccines and compositions of this invention are the well-known specific or non-specific proteolytic, dissociative or enzymatic methods. The particular method chosen depends on the precursor and the level of conversion desired for a particular composition or vaccine.

Illustrative of the above-described compositions that are useful as sources of the anti-HBe eliciting compositions of this invention are the various HBeAgs and HBcAgs isolatable from the serum of HBV infected patients or from Dane particles isolated from that serum. For example, it has been reported that HBeAgs are released from HBV core particles purified from serum [Takahashi et al., J. Immunol., 17, pp. 102-105 (1976)] or similar particles purified from liver [Budkowska et al., J. Immunol., 123, pp. 1415-1416 (1979); Yoshizawa et al., J. Gen. Virol., 42, pp. 513-519 (1979)] by treatment with pronase [Budkowska et al.], pronase and 2-mercaptoethanol (2ME) [Takahashi et al.], or sodium dodecyl sulphate (SDS) and 2-mercaptoethanol [Budkowska et al.; Takahashi et al.; Yoshizawa et al.] or by disruption by sonication and by treatment with chaotropic agents or centrifugation in CsCl [H. Ohori et al., "Antigenic Conversion From HBcAg To HBeAg by Degradation Of Hepatitis B Core Particles", Intervirology, 13, pp. 74-82 (1980)]. However, because of the problems associated with serum-derived vaccines these sources are not preferred for the vaccines and compositions of this invention.

Preferably, the compositions useful in this invention are prepared by the fermentation of appropriate hosts that have been transformed with and express a DNA sequence encoding a polypeptide displaying the antigenicity of HBcAg [e.g., Burrell et al, Nature, 279, pp. 43-47 (1979); Pasek et al., Nature, 282, pp. 575-79 (1979)] or hosts transformed with and expressing a DNA sequence encoding a polypeptide displaying the antigenicity of one of the HBeAgs [e.g., European patent application 75,393]. Also preferred are hosts transformed by DNA sequences that encode COOH-truncated HBcAgs. These preparations also contain e antigen activity. Finally preferred are synthetic peptides that display the antigenicity of HBeAgs or which are precursors of such HBeAg activity. These compositions are preferred because they do not require isolation of components from the serum of infected patients and accordingly pose no risk of active infection to workers during preparation of those vaccines or to patients treated with them.

More preferably, the compositions of our invention are prepared from fermentation extracts of hosts transformed with and expressing a DNA sequence encoding at least one polypeptide displaying the antigenicity of HBcAg because those extracts contain high levels of readily extractable polypeptides displaying the antigenicity of HBcAg.

Most preferably, these HBcAg-containing extracts are treated by one of the methods described in European patent application 75,393 or similar methods before use. Such methods include, for example, preparing a bacterial extract of a host characterized by the expression of a polypeptide displaying the antigenicity of hepatitis B virus core antigen and digesting said extract with a reducing agent-resistant protease in the presence of a reducing agent. This treatment converts a part or all of the HBcAg of the extract into polypeptides displaying HBeAg antigenicity. Alternatively, with more concentrated and purified bacterial extracts, a similar conversion of at least a portion of the polypeptides displaying the antigenicity of HBcAg to polypeptides displaying the antigenicity of HBeAg may be effected by a reducing agent under dissociating conditions, e.g., SDS or like conditions. Finally, SDS alone may be used to effect the total or partial conversion.

In the above-described methods, the reducing agent-resistant proteases may be selected from any of the wide variety of such known proteases, such as pronase, subtilisin, papain, chymotrypsin, bromelin,

trypsin, thermolysin, protease κ, carboxypeptidase A or carboxypeptidase B. In like manner, the reducing agents may be selected from any of the well known reducing agents, such as 2-mercaptoethanol (2ME), dithiothreitol (dTT), dithioerythritol, thioglycollate, glutathione or sodium borohydride. The amounts of the various reagents to convert the HBcAgs, partially or totally, to HBeAgs may be determined by those of skill in the art by simple serological tests. We believe that these conversion processes may result in the formation of a population of HBcAg degradation products, many of which exhibit HBeAg activity. These active products may therefore represent the various serological variants of HBeAg.

To illustrate the wide variety of compositions and treatments that produce other compositions that elicit anti-HBe formation, we have depicted in Table I the serum analyses of a series of rabbits treated with a variety of such compositions.

## TABLE I

| Rabbit[1] | Anti-HBc[2] | | | Anti-HBe[2] | Composition |
|---|---|---|---|---|---|
| | 1:10 | 1:30 | 1:100 | 1:5 | |
| 21 | 7.2 | 5.4 | 6.0 | 6.3 | Particulate extract from strain R1-11 (infra) (~80% HBcAg); some proteolytic degradation products were observed in the composition by gel analysis |
| 22 | 9.2 | 6.9 | 6.6 | 2.7 | |
| 23 | 5.8 | 6.9 | 6.9 | 10.6 | |
| 24 | 7.0 | 7.6 | 5.8 | 9.9 | Same as above, except the composition was was treated with 1% SDS and 20mm 2ME |
| 25 | 6.5 | 5.9 | 4.6 | 4.3 | |
| 41 | 8.3 | 6.4 | 4.8 | 5/6 | Partially purified HBcAg preparation from strain RI-11, treated with 1% SDS and 20mm 2ME |
| 42 | 6.6 | 6.8 | 5.8 | 5.0 | |
| 43 | 6.6 | 5.7 | 5.2 | 3.5 | Partially purified HBcAg preparation from strain RI-11, digested with trypsin |
| 44 | 7.9 | 5.0 | 2.8 | 3.6 | |
| 45 | 8.2 | 4.5 | 2.6 | 3.5 | Same as 43-44, except the digest was treated with 1% SDS and 20mm dT. |
| 46 | 7.9 | 5.9 | 4.3 | 2.1 | |
| 47 | 11.3 | 6.9 | 5.0 | 3.2 | Same as 43-44, except the digest was fractionated to isolate the 17K major peak |
| 48 | 7.0 | 7.6 | 7.7 | 10.7 | |
| 49 | 6.9 | 6.2 | 4.0 | 5.3 | Same as 47-48, except the 17K major peak was treated with 1% SDS |
| 50 | 7.4 | 6.7 | 5.4 | 9.0 | |

---

[1] Each rabbit was treated by three inoculations of about 200 µg of the specified composition in incomplete Freund's adjuvant. The injections in rabbits 21-25 were three weeks and then two weeks apart. In the other rabbits the injections were 2 weeks and then 6 weeks apart.

[2] Negative/Positive (N/P) ratios.

## TABLE I (cont'd)

| Rabbit[1] | Anti-HBc[2] | | | Anti-HBe[2] | Composition |
|---|---|---|---|---|---|
| | 1:10 | 1:30 | 1:100 | 1:5 | |
| Negative Control | 1.0 | -- | -- | 1.0 | |
| Positive Control | 8.6 | -- | -- | 10.8 | (Human serum) |
| 68 | -- | -- | -- | 4.2 | Partially purified HBcAg from strain RI-11, treated with 1% SDS, 2ME |
| 69 | -- | -- | -- | 11.7 | Crude extract HBcAg from strain HBcAg-Tac in 30% $(NH_4)_2SO_4$ |
| 71 | -- | -- | -- | 8.4 | Same as 69, except treated with 1% SDS and 2ME |

---

[1]    Each rabbit was treated by three inoculations of about 200 µg of the specified composition in incomplete Freund's adjuvant.  The injections in rabbits 21-25 were three weeks and then two weeks apart.  In the other rabbits the injections were 2 weeks and then 6 weeks apart.

[2]    Negative/Positive (N/P) ratios.

A wide variety of host and expression vectors may be employed to produce the above-described fermentation extracts. For example, any of the well-known expression vectors may be employed. These include vectors derived from segments of chromosomal, non-chromosomal and synthetic DNA sequences, such as various known derivatives of SV40 and known bacterial plasmids, e.g., plasmids from E. coli including col El, pCRl, pBR322, pMB9 and their derivatives, wider host range plasmids, e.g., RP4, phage DNAs, e.g., the numerous derivatives of phage λ, e.g., NM 989, and other DNA phages, e.g., M13 and Filamentous single-stranded DNA phages and vectors derived from combinations of plasmids and phage DNAs such as plasmids which have been modified to employ phage DNA or other expression control sequences or yeast plasmids such as the 2 μ plasmid or derivatives thereof.

These vectors may be characterized by various expression control sequences. These, for example, include the operator, promoter and ribosome binding and interaction sequences (including sequences such as the Shine-Dalgarno sequences) of the lactose operon of E. coli ("the lac system"), the corresponding sequences of the tryptophan synthetase system of E. coli ("the trp system"), the major operator and promoter regions of phage λ ($O_L P_L$ as described above and $O_R P_R$), a control region of Filamentous single-stranded DNA phages, or other sequences which control the expression of genes of prokaryotic or eukaryotic cells and their viruses or combinations thereof.

In like manner, any of the well-known hosts may be employed. These include, for example, bacterial hosts such as E. coli HB 101, E. coli X1776, E. coli X2282, E. coli MRCI and strains of Pseudomonas, Bacillus subtilis, Bacillus stearothermophilus and other bacilli, streptomyces, yeasts and other fungi, animal or plant hosts such as animal (including human) or plant cells in culture or other hosts. Of course, not all host/vector combinations may be equally efficient.

While not all host/vector combinations may function with equal efficiency for the expression of a given DNA sequence, the particular selection of host/cloning vehicle combination may be made by those of skill in the art using well recognized principles without departing from the scope of this invention.

It should also be understood that the vaccines and compositions of our invention may include other components, in addition to the above-described anti-HBe eliciting components. These other components, for example, may elicit the formation of anti-HBc or anti-HBs, or both, in treated patients. For example, one preferred embodiment of our invention is characterized by compositions that elicit the formation of anti-HBc, in addition to the above-described effective titers of anti-HBes. However, it is a surprising and important attribute of this invention that anti-HBs eliciting components are not needed in the vaccines, compositions or methods of this invention to provide protection against HBV. In view of the limited supply of HBsAg and insensitivity of some patients to HBsAg (supra), this is a marked and very important advantage of our invention.

The compositions of this invention may also include other components or be subject to other treatments during preparation to enhance their immunogenic character or to improve their tolerance in patients. For example, various pharmaceutically acceptable adjuvants and other compounds routinely employed in vaccines may be employed. At present, we prefer Alum. In addition, various treatments, such are those that result in micell formation, may be usefully employed in conjunction with the preparation of the vaccines and compositions of this invention.

The vaccines and compositions of this invention may be administered by subcutaneous or intramuscular injection. Preferably, intramuscular injection is used. The frequency of administration will depend on the particular composition of the vaccine and the patient to be treated. However, it is believed that one or two doses, one month apart, followed by a booster at six months to one year will be sufficient. The dosage will depend on the size and condition of the patient treated. And, the need for any subsequent dose or booster will depend on the level of anti-HBe titer that results from the initial immunizations.

In order that this invention may be more fully understood, the following examples are set forth. These examples and their conditions and procedures are for illustrative purposes only.

## Example 1

This is an example of the use of bacterial extracts of hosts transformed and expressing polypeptides displaying HBcAg antigenicity in the compositions of this invention.

In this example *E. coli* K12 strain HB101 [H. Boyer and D. Roulland-Dussoix, *J. Mol. Biol.*, 41, pp. 459-72 (1969)] transformed with plasmid pHBV-RI-11 was employed to produce a polypeptide displaying the antigenicity of HBcAg. This polypeptide was then employed as a source of the anti-HBe eliciting components that characterize the vaccines, compositions and methods of this invention.

Plasmid pHBV-RI-11 which expresses a DNA sequence coding for a polypeptide displaying the antigenicity of HBcAg in an appropriate host was constructed as follows. One liter of a culture of Escherichia coli K-12 strain HB101 harboring the plasmid pHBV139A [M. Pasek et al., Nature, 282, pp. 575-579 (1979)], encoding the entire HBcAg gene on a fragment excisable by PstI, was grown to O.D.$_{1550}$=1.0, and the plasmid isolated from the harvested cells as described by D. Clewell, J. Bacteriol., 110, pp. 667-676 (1972). 60 µg of purified pHBV139A were digested with 5 units of PstI overnight at 37°C in 10 mM Tris-HCl (pH 7.6), 5 mM MgC$_2$, 1 mM dithiothreitol (dTT) and 50 mM NaCl. The digested plasmid was electrophoresed on a preparative 8% polyacrylamide gel and the excised DNA fragment containing the HBcAg coding sequence recovered from the gel by UV-shadowing and elution as described by A. Maxam and W. Gilbert, Methods Enzymol., 65, pp. 499-560 (1980). The ends of the isolated DNA fragment were digested with exonuclease BAL 31 in 0.6 ml 20 mM Tris-HCl (pH 8), 12 mM CaCl$_2$, 12 mM MgCl$_2$, 60 mM NaCl and 1 mM ethylenediamine tetraacetic acid (EDTA). The reaction was stopped by extraction with an equal volume of phenol. 0.2 µg of EcoRI or HindIII linkers (encoding the recognition site of the restriction enzymes EcoRI or HindIII, respectively) were phosphorylated by 1 unit of polynucleotide kinase in 10 µl volume in the presence of adenosine triphosphate (ATP) and γ-32 ℓ-ATP in a ratio of 5:1, as described by A. Maxam and W. Gilbert, Methods Enzymol., 65, pp. 499-560 (1980).

The phosphorylated and labelled linkers were ligated to 0.2 µg of the BAL 31-digested fragment with 1 unit of ligase for 1 h at 15°C in 20 ml of ligation buffer; 50 mM Tris-HCl (pH 7.6), 10 mM MgCl$_2$, 10 mM DTT and 1 mM ATP. The ligation was stopped by phenol extraction, as above, and the product digested overnight at 37°C by 10 units of EcoRI or HindIII (as appropriate) and 10 units of BamHI with the addition of 90 µl of buffer: 10 mM Tris-HCl (pH 7.5), 50 mM NaCl, 5 mM MgCl$_2$, 1 mM dTT and 0.15% Triton X-100. The sample was centrifuged for 1 min in a desktop clinical centrifuge through 2 ml of Sephadex G-50 in 10 mM Tris-HCl (pH 8), 1 mM EDTA. 0.2 µg of pExlac150, a cloning vehicle with an EcoRI or a HindIII restriction site suitable for expressing cloned genes, constructed by H. Weiher, Dissertation, University of Heidelberg (1980) and isolated as described for pHBV139A above, were digested with 1 unit of EcoRI or HindIII and BamHI in 10 µl of buffer, as described above. The above-prepared fragment was then inserted into the cloning vehicle by incubation with 1 unit of ligase in 20 µl of ligation buffer for 4 h at room temperature.

Escherichia coli K-12 strain HB101 was then transformed with the above-prepared recombinant plasmid, as described by M. Mandel and A Higa, J. Mol. Biol., 53, pp. 159-162 (1970). Transformants were selected on rich agar plates by resistance to 50 µg/ml ampicillin and screened for the expression of polypeptides displaying the antigenicity of HBcAg by the method of S. Broome and W. Gilbert, Proc. Natl. Acad. Sci. (USA), 75, pp. 2746-2749 (1978), as described by C. Burrell et al., Nature, 279, pp. 43-48 (1979).

The recombinant plasmid contained by one of the hosts expressing those polypeptides, designated pHBV-RI-11 (it contains an EcoRI linker), was sequenced by the method of A. Maxam and W. Gilbert, Methods Enzymol., 65, pp. 499-560 (1980) at the region of fusion between the HBV DNA and the β-galactosidase gene of pEXlac150. The nucleotide sequence demonstrates that amino acid 8 of β-galactosidase is fused to amino acid 3 of HBcAg by 3 amino acids encoded by the EcoRI linker.

Products displaying the antigenicity of HBcAg were then prepared by culturing E. coli Kl2 HB101 transformed with pHBV-RI-11 to produce a product that aggregates to form particles resembling native HBV core particles that are isolated from human liver [S. Stahl et al., "Hepatitis B Virus Core Antigen, Its Synthesis In Escherichia coli And Application In Diagnosis", Proc. Natl. Acad. Sci. (USA), 79, pp. 1606-10 (1981) and Richmond and Cohen, Nature, 296, pp. 667-68 (1982)].

Extracts of these fermentation cultures were then employed to prepare both substantially homogeneous samples of polypeptides displaying the antigenicity of HBcAg and somewhat less extensively purified preparations by combinations of ultra-centrifugation, exclusion chromatography and other purification steps. These preparations elicited both anti-HBe and anti-HBc in rabbits. See, e.g., Table I, supra. We believe that the presence of the anti-HBe indicates that during fermentation or purification, or perhaps by in vivo metabolism, these preparations of HBcAgs were converted at least to some extent into compositions containing polypeptides displaying HBeAg antigenicity. These preparations were shown to be pyrogen-free by the limulus test.

To prepare compositions in accordance with this invention for inoculation into chimpanzees, the acknowledged test animal for the study of HBV and the demonstration of protection against HBV infection, we employed a fermentation culture of E. coli Kl2 HB 101 (pHBV-RI-11). As described above, this strain produces a polypeptide displaying HBcAg specificity.

To isolate these polypeptides from the fermentation culture and to purify them a wide variety of combinations of chromatographic, filtration, extraction and precipitation steps may be employed. All are well-known purification techniques.

For example, we have prepared a composition of these polypeptides from a fermentation extract of such strain by low speed centrifugation to remove cellular debris (10000 rpm, 10 min), precipitation with $(NH_4)_2SO_4$ (55% saturation), solubilization of the precipitate in PBS buffer, dialysis, fractionation on a Sepharose 4B column and isolation of the HBcAg-active fractions by precipitation with $(NH_4)_2SO_4$, solubilization with PBS and dialysis as before.

It should, however, be understood that more or less extensive purification schemes may also be employed in preparing the compositions of this invention, because both substantially homogeneous, as well as more crude preparations of polypeptides displaying HBcAg antigenicity are useful in the compositions and methods of this invention. All that is necessary is that there be a sufficient amount of an active anti-HBe eliciting component, or precursor thereof, in the preparation to allow the formation of a sufficient titer of anti-HBe in the treated patient. In this regard, it may be preferable to use crude preparations of HBcAg because these preparations already contain as degradation products high levels of anti-HBe eliciting components.

For inoculation, we employed various preparations prepared by the purification schemes discussed above (these contained approximately 80% HBcAg), dissolved them in PBS buffer and mixed them overnight with an equal volume of 1 M $NH_4HCO_3$ and with a volume equal to the resulting total volume of 10% Alum at 4°C. After centrifugation of the suspensions (2000 rpm, 10 min, 4°C), we washed the pellets with the same solution of $NH_4HCO_3$ and 10% Alum, described above, recentrifuged them and dispersed the pellets in PBS to a total volume of 2-2.5 ml. These preparations may be stored at -70°C.

For inoculation, we thawed the above-described preparations and used samples, containing 100 µg of HBcAg, to inoculate intramuscularly two chimpanzees:*

| Chimpanzees | Sex | Date of Birth | Body Weight |
|---|---|---|---|
| Gwen | F | August 8, 1972 | 47 Kg |
| Rinka | F | August 27, 1971 | 52 Kg |

Gwen received two inoculations of the compositions of this invention 4 months apart. Rinka received three injections of the compositions of this invention, the first two 3 weeks apart and the third 4 months after the second. Each received the same composition at about the same time.

We also used two chimpanzees who had no history of exposure to HBV as controls:

| Chimpanzees | Sex | Date of Birth | Body Weight |
|---|---|---|---|
| Peter | M | January 26, 1978 | 28 Kg |
| Frankje | F | April 6, 1976 | 34 Kg |

We challenged the four chimpanzees with an intravenous inoculation of $5 \times 10^3$ infectious doses of HBV serotype ayw (a gift of R. H. Purcell). Chimpanzees Gwen and Rinka were challenged by the virus 30 days after their last injection of the compositions of this invention.

---

* We used this high level of HBcAg because we wanted to insure that our preparations also contained sufficient HBe to develop effective titers of anti-HBe. Lower levels may also be used depending on the concentration of anti-HBe eliciting components present. We did not measure the actual concentration of HBeAgs in our inoculums because such determination is difficult in the presence of HBcAg.

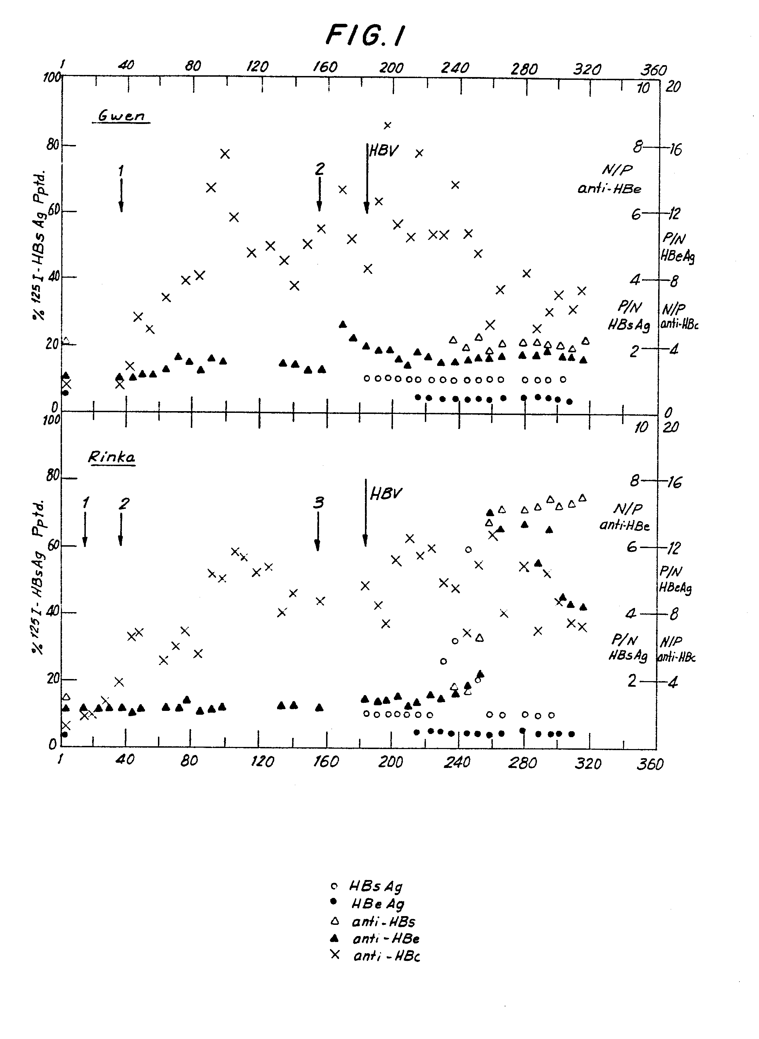

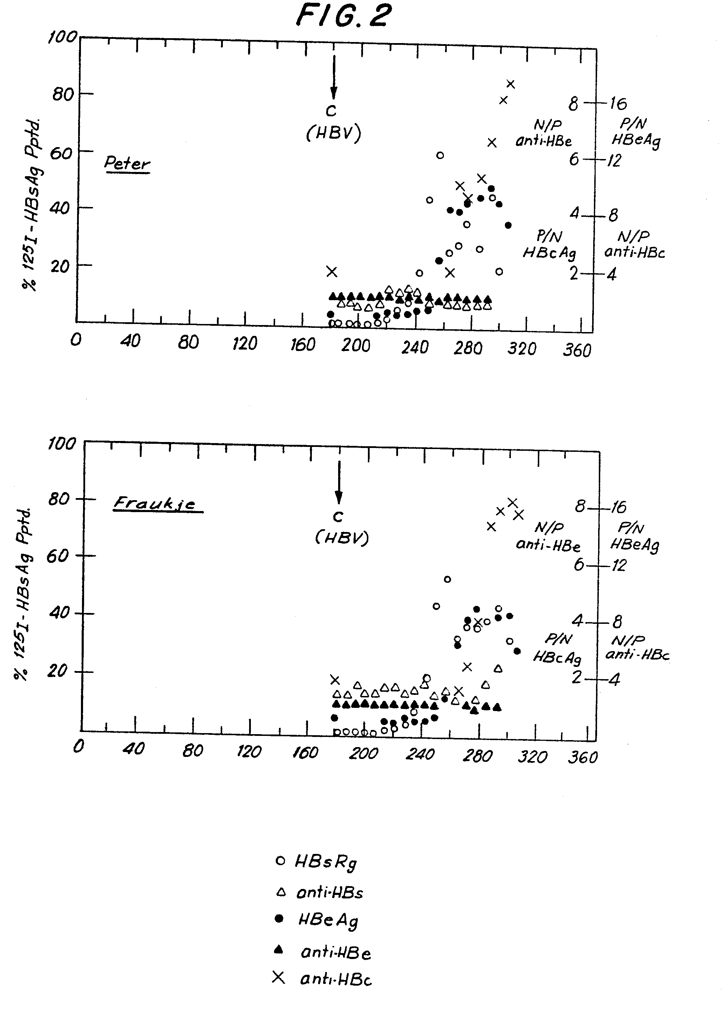

Referring now to Figures 1 and 2, we have displayed therein the serum conversion analyses (HBsAg, HBeAg, anti-HBs, anti-HBe and anti-HBc) for serum samples taken weekly from the four chimpanzees used in this example.

As shown in Figure 1, the two chimpanzees inoculated with compositions of this invention, as described above, had different responses. Rinka immediately developed an observable anti-HBc titer, but no observable anti-HBe titer. Gwen immediately developed an anti-HBc titer, similar to Rinka's, and a low but definite anti-HBe titer. Both animals maintained high anti-HBc titers for several months. These were not significantly increased by an additional inoculation of our preparation after an interval of 4 months. Gwen's anti-HBe titer increased somewhat after the second injection, but Rinka still had no observable anti-HBe titer after that injection.

As also shown in Figure 1, after challenge by HBV serotype ayw, Gwen, the chimpanzee with the observable anti-HBe titer, displayed no symptoms of infection and developed neither antigenemia nor any significant change in antibody levels.* Subsequently, Gwen began to develop an anti-HBs titer (not shown in Figure 1).

After challenge by HBV serotype ayw, Rinka, the chimpanzee with no observable anti-HBe titer had a very mild and short-lived HBV infection. The lack of virulence and short time span of this infection indicates that only a brief burst of viral replication occurred in Rinka. As shown in Figure 1, this burst of replication led to moderate synthesis

---

*   Over the past several years of vaccine evaluations at the Primate Center, TNO, the Netherlands, where these tests were conducted, none of the control animals (23) remained uninfected upon inoculation with HBV.

of HBsAg, but no synthesis of HBeAg and a rapid boost
in anti-HBs, and particularly anti-HBe.  As shown
in Figure 2, both control chimpanzees developed virulent HBV infections.

These results demonstrate unexpectedly
and surprisingly that the anti-HBe elicited by the
compositions of this invention, even in the absence
of anti-HBs, is protective against HBV infection,
or at lower levels (Rinka) at least lessens the virulence and course of the HBV infection.  For example,
Gwen, who had a low, but detectable, level of anti-
HBe after treatment with compositions of this invention, was completely protected from HBV infection
and viral replication.  Rinka, on the other hand,
after treatment with compositions of this invention,
initially had no observable level of anti-HBe.  However, in view of the rapid rise observed in Rinka's
anti-HBe titer and the absence of HBeAg synthesis,
after challenge, we believe that Rinka initially
had a low, albeit not observable, titer of anti-HBe
elicited by the compositions of this invention.
This low titer, while insufficient to protect Rinka,
did markedly lessen the severity of Rinka's subsequent
HBV infection.

## Example 2

This is an example of the use of SDS or
SDS and 2-mercaptoethanol (2 ME) to raise the concentration of active anti-HBe eliciting components in
compositions of this invention prepared from bacterial
extracts of hosts transformed and expressing polypeptides displaying HBcAg antigenicity.

In this example, we employed HBcAg preparations from E. coli K12 HB101 (pHBV-RI-11) similar
to those described in Example 1.  However, in this
example, we treated the preparations (in PBS) with
either 1% SDS or 1% SDS and 1% 2-mercaptoethanol at 37°C for some period of time (2h to a few minutes) to convert at least a portion of the HBcAg activities in those extracts to HBeAg activities [see, e.g., European patent application 73,395]. We then diluted the preparation with PBS to 0.1% SDS before inoculation.

For inoculation we again used preparations containing 100 µg of HBcAg. We did not use Alum because of the presence of the SDS. Because of the above-described treatment, these samples contained higher relative concentrations of HBeAg. Chimpanzees inoculated with these preparations have developed high titers of anti-HBe. These titers are more than twice that observed in Gwen. Because of the level of these titers, we expect that the chimpanzees are protected from HBV.

We subsequently inoculated these chimpanzees with a booster (100 µg HBcAg/Alum) prepared as follows: HBcAg, prepared substantially as described above except under the control of a TAC promoter (supra), was digested with trypsin (5% w/w) in 4.5 M urea to convert HBcAg to HBeAg in the absence of SDS. The reaction digest was then fractionated by gel filtration (Sephacryl 5200 (Pharmacia) in bicarbonate buffer (pH 9.4)). After dialysis into PBS, we precipitated the mixture onto Alum, as before. This booster resulted in higher titers of anti-HBe in both chimpanzees. We then challenged the chimpanzees, as before, with hepatitis B virus.

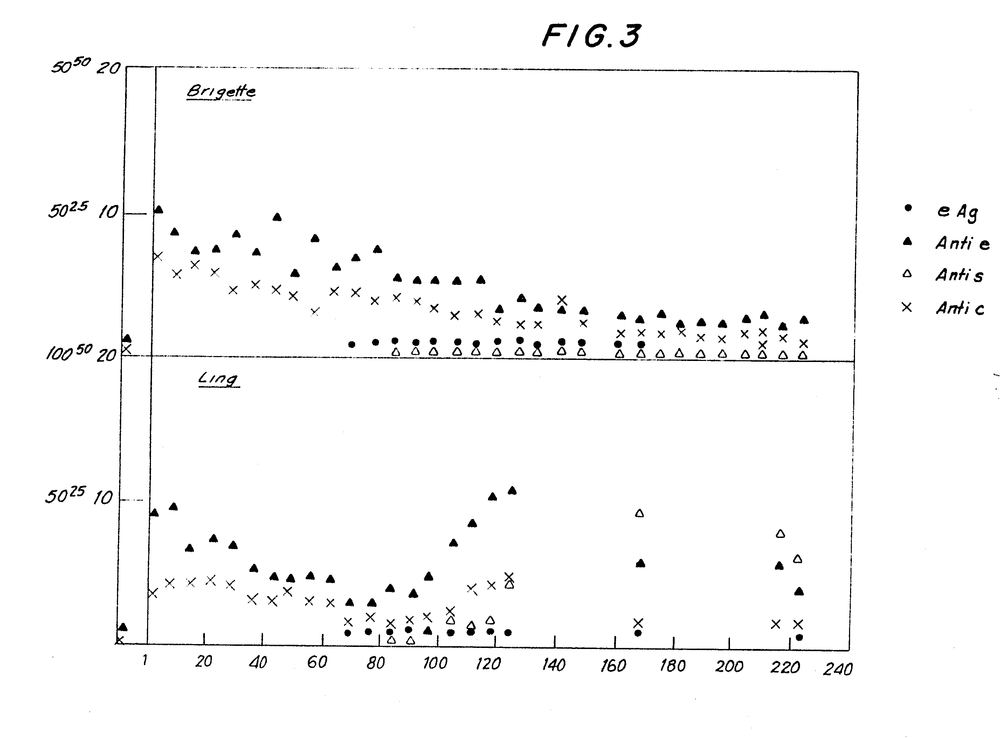

Referring now to Figure 3, we have depicted therein the serum conversion analyses (HBeAg, anti-HBs, anti-HBe and anti-HBc) for serum samples taken weekly from these two animals (A and B) after virus challenge. As depicted in Figure 3, chimpanzee A's titers of anti-HBc and anti-HBe decreased slowly during the several months after challenge. However, chimpanzee A never developed HBsAg (not shown) or anti-HBs titers demonstrating that it had been protected from HBV by our composition. Chimpanzee B's
titers of anti-HBc and anti-HBe also decreased slowly
after challenge. However, at about 100 days after
challenge, there was a boost in anti-HBe titer and
the appearance of a very low and short-lived titer
(about 8 days) of HBsAg (not shown). Subsequently,
no HBsAg titer could be observed, but anti-HBs
appeared. These results, like those of Rinka, above,
demonstrate that the compositions of this invention
partially protected chimpanzee B from hepatitis B
viral infection.

While we have hereinbefore presented a
number of embodiments of this invention, it is
apparent that our basic construction can be altered
to provide other embodiments which utilize the process
of this invention. Therefore, it will be appreciated
that the scope of this invention is to be defined
by the claims appended hereto rather than the specific
embodiments which have been presented hereinbefore
by way of example.

We claim:

1.    A pharmaceutically acceptable composition that is characterized by a component that elicits in a treated patient the formation of antibodies to hepatitis B viral e antigens at a titer effective to protect the patient for some period of time against hepatitis B viral infection or at a titer effective to lessen the severity of a hepatitis B viral infection in that patient.

2.    The composition of claim 1, characterized by the absence of HBsAg or precursors thereof.

3.    The composition of claim 1 or 2, wherein the titer is effective to protect the treated patient for some period of time against hepatitis B viral infection.

4.    The composition of claims 1 to 3, wherein the component that elicits the formation of said anti-HBe titer is selected from the group consisting of pharmaceutically acceptable compositions that contain from 100% to the lowest concentration of any of the serological variants of HBeAg that is effective after one or more treatments of a patient to elicit said titer of anti-HBe.

5.    The composition of claim 4, wherein said pharmaceutically acceptable compositions are prepared from the fermentation extracts of a host transformed with and expressing a DNA sequence selected from the group consisting of sequences encoding at least one polypeptide displaying the antigenicity of HBcAg and sequences encoding at least one polypeptide displaying the antigenicity of HBeAg.

6.    The composition of claim 5, wherein said fermentation extract of a host transformed with and expressing a DNA sequence encoding a polypeptide displaying the antigenicity of HBcAg is treated enzymatically or proteolytically to convert at least a portion of the polypeptides displaying the antigenicity of HBcAg into polypeptides displaying the antigenicity of HBeAg.

7. The composition of claim 6, wherein said treatment is in vitro and is selected from the group consisting of treatments with reducing agent-resistant proteases in the presence of a reducing agent, reducing agents under dissociating conditions and sodium dodecylsulfate.

8. The composition of claim 4, wherein said pharmaceutically acceptable compositions are prepared from one or more synthetic polypeptides displaying the antigenicity of HBeAgs.

9. The compositions of any one of claims 1-8, wherein said compositions also elicit the formation of antibodies to HBcAg in the treated patient.

10. A method for treating patients against hepatitis B viral infections characterized by the step of treating said patients with a composition defined in claims 1-9.

11. The method of claim 10, wherein said method protects the treated patients for some period of time against hepatitis B viral infection.

FIG.1

FIG. 2

0133599

2/3

# FIG.3

Legend:
- ● e Ag
- ▲ Anti e
- △ Anti s
- ✕ Anti c

0133599